# EUROPEAN PATENT SPECIFICATION

(11) **EP 1 558 597 B1**
(45) Date of publication and mention of the grant of the patent: **07.05.2008**
(21) Application number: 02765680.0
(22) Date of filing: 18.09.2002
(51) Int. Cl.: C07D 307/32

(54) **CONTINUOUS PROCESS FOR THE PRODUCTION OF OPTICALLY PURE (S)-BETA HYDROXY-GAMMA-BUTYROLACTONE**
KONTINUIERLICHES VERFAHREN ZUR HERSTELLUNG VON OPTISCH REINEM (S)-BETA HYDROXY- GAMMA-BUTYROLACTON
PROCEDE CONTINU POUR LA PRODUCTION DE (S)-BETA HYDROXY-GAMMA-BUTYROLACTONE DE PURETE OPTIQUE

(43) Date of publication of application: 03.08.2005
(73) Proprietor: SK Holdings Co., Ltd., Seoul 110-110 (KR)
(72) Inventor: KWAK, Byong-Sung, 305-761 Daejeon (KR); CHUNG, Ki-Nam, 305-761 Daejeon (KR); KIM, Tae-Yun, 305-728 Daejeon (KR); KOH, Ki-Ho, 305-728 Daejeon (KR); KIM, Jin-Woong, 305-761 Daejeon (KR); LEE, Sang-Il, 305-728 Daejeon (KR)
(74) Representative: Knowles, James Atherton
(86) International application number: PCT/KR2002/001775
(87) International publication number: WO 2004/026223

(56) References cited:
- WO-A1-02/10147
- JP-A- 4 266 881
- JP-A- 6 172 256
- US-B1- 6 355 848
- HAMADA Y ET AL: "An Efficient Synthesis of C20-C25 Building Blocks for Calyculin A" TETRAHEDRON, ELSEVIER SCIENCE PUBLISHERS, AMSTERDAM, NL, vol. 52, no. 24, 10 June 1996 (1996-06-10), pages 8297-8306, XP004103878 ISSN: 0040-4020
- TANAKA A. ET AL: 'A novel synthesis of (R)-and (S)-4-hydroxytetrahydrofuran-2-ones' SYNTHESIS no. 6, 1987, pages 570 - 573, XP001026455

## Description

### TECHNICAL FIELD

The present invention relates to a continuous process for the production of chemically pure (S)-β-hydroxy-γ-butyrolactone having desired optical activity by hydrogenation of carboxylic acid ester derivative.

### PRIOR ART

Optically pure substituted γ-butyrolactone has been variously used as intermediates for production of pharmaceuticals, agrochemicals, flavors and fragrances, such as L-carnitine, ECHB (ethyl (S)-4-cyano-3-hydroxybutyrate), (S)-1,2,4-butanetriol, etc. (U.S. Pat. No. 5,473,104).

Various techniques for the preparation of (S)-β-hydroxy-γ-butyrolactone are described in the prior art literatures. In this regard, U.S. Pat. Nos. 5,292,939, 5,319,110, and 5,374,773 disclose a process for preparing a substituted γ-butyrolactone by oxidation of a water-soluble carbohydrate reactant. Such processes, however, are disadvantageous in that only a low concentration of the reactant should be employed during the oxidation due to reaction heat. Moreover, except a chromatography technique, any isolation methods for isolation of a reaction product are not discussed. Further, the yield of the product is not also mentioned. Consequently, the above processes are unsuitable for large-scaled preparation.

For the preparation of a substituted γ-butyrolactone compound, there has been reported a multi-step procedure in which L-malic acid or L-aspartic acid is used as a starting material (J. Org. Chem. 1981, 46, 4319, Synth. Commun. 1986, 16, 183). However, this technique has a significant problem that an optical activity is not maintained in a reaction intermediate generated during the reaction procedure. Further, this process is difficult to be applied on industrial scale.

Meanwhile, there was reported another process which involves a reduction of (S)-malic acid ester derivative as a starting material by use of borane-dimethylsulfide and sodium borohydride (Chem. Lett. 1984, 1389). This method, however, is disadvantageous in that high preparation cost is needed due to a batch type reaction. Moreover, the inevitable generation of large amounts of waste makes this process environmentally harmful and unsuitable for industrial use.

In U.S. Pat. No. 5,808,107, there is disclosed a process for producing (S)-β-hydroxy-γ-butyrolactone by reducing L-malic acid dimethyl ester with lithium chloride and sodium borohydride, to afford (S)-3,4-dihydroxybutyric acid, which is then treated with an acid (HCI) in a methanol solvent. According to the above patent, the optical purity is maintained during the reaction. However, this process is environmentally harmful, and has shortcomings attributable to a complicated batch type preparation method. Furthermore, the use of high-priced explosive reducing agent makes the process unfeasible in the aspect of cost. Hence, the above process is unsuitable for preparation on a large scale. Also, ether used as a reaction solvent is harmful to the human body when used in large amounts, and is explosive.

In U.S. Pat. No. 5,998,633, there is disclosed a production process for the preparation of a substituted γ-butyrolactone by oxidizing a carbohydrate, yielding an acetonide intermediate, which is treated with an inorganic acid (HCI aqueous solution). This technique suffers from a complexity of the reaction mechanism and the generation of large quantities of waste, and thus is difficult to apply industrially.

WO-A-0210147 relates to a process for the production of optically pure (S)-beta-hydroxy-gamma-butyrolactone in a continuous manner by hydrogenation of substituted carboxylic acid derivatives in a fixed bed reactor which is filled with a catalyst comprising a noble metal and a support.

In U.S. Pat. No. 6,221,639 there is disclosed a method for the production of (S)-β-hydroxy-γ-butyrolactone having high optical purity, comprising reacting amylopectin using an enzyme to afford an oligosaccharide which is then reacted with an alkaline anionic exchange resin and an oxidizing agent, to obtain (S)-3,4-dihydroxy-butyric acid, which is desorbed and subjected to esterification and cyclization. However, this method is disadvantageous in terms of low preparation yield due to a complexity of the reaction mechanism, and high cost is problematic when applied on the large scale.

As stated above, the conventional preparation methods adopt a batch type process using a solid or liquid reagent such as an oxidizing agent or a reducing agent, which makes the production efficiency unfavorable. In particular, a large quantity of waste is generated during the process and a complexity of the process is a major cause of low preparation yield. Accordingly, such methods are unsuitable for the preparation of a large scale and are limited in their industrial applications.

### DISCLOSURE OF THE INVENTION

Leading to the present invention, the intensive and thorough research into continuous production processes of (S)-β-hydroxy-y-butyrolactone, carried out by the present inventors aiming to solve the problems encountered in the prior arts, led to the development of a catalyst capable of being easily prepared and increasing production efficiency of the target product, by which a production yield can be increased, and to the development of a reaction system capable of retaining a desired optical purity.

Therefore, it is an object of the present invention to provide a continuous process for efficiently producing chemically pure (S)-β-hydroxy-γ-butyrolactone while retaining an optical activity to a desired level from optically pure carboxylic acid ester derivative employed as a starting material, by which the simplicity of the.process and the environmental safety can be assured.

In accordance with an embodiment of the present invention, there is provided a continuous process for the production of chemically pure (S)-β-hydroxy-γ-butyrolactone having desired optical activity, which comprises:
a) dissolving carboxylic acid ester derivative having the following Formula 2 in solvent at an amount of 2-50 wt%, the solvent being added with an organic or inorganic acid; 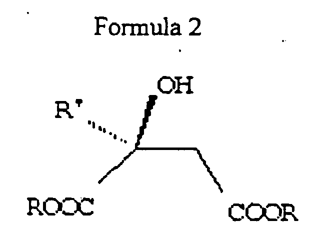
b) subjecting the carboxylic acid ester derivative solution to hydrogenation at 50-500 °C under a pressure of 1.0-379.2 bar (15-5,500 psig) at weight-hourly-space-velocity of 0.1-10 h⁻¹, in a fixed bed reactor charged with a metal catalyst-impregnated inorganic oxide support, a molar ratio of hydrogen to carboxylic acid ester derivative ranging from 2 to 10; and
c) recovering (S)-β-hydroxy-γ-butyrolactone having the following Formula 4 from the hydrogenation products, 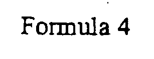 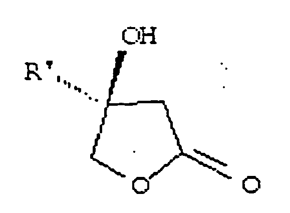 wherein R represents linear or cyclic alkyls, or aryl groups, of from 1 to 10 carbon atoms, and R' represents hydrogen or methyl.

In accordance with a preferred embodiment of the present invention, there is provided a continuous process for the production of chemically pure (S)-β-hydroxy-γ-butyrolactone having desired optical activity, which comprises:
a) dissolving carboxylic acid ester derivative having the following Formula 2 in solvent at an amount of 2-50 wt%, the solvent being added with an organic or inorganic acid; 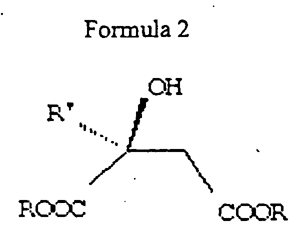
b) subjecting the carboxylic acid ester derivative solution to hydrogenation at 50-500 °C under a pressure of 1.0-379.2 bar (15-5,500 psig) at weight-hourly-space-velocity of 0.1-10 h⁻¹, in a fixed bed reactor charged with a metal catalyst-impregnated inorganic oxide support to give hydrogenation products containing an intermediate having the following Formula 3 and (S)-β-hydroxy-γ-butyrolactone having the following Formula 4, a molar ratio of hydrogen to carboxylic acid ester derivative ranging from 2 to 10; and 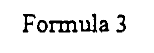 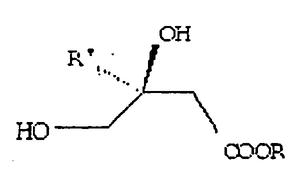 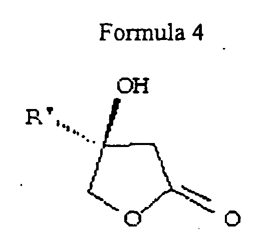
c) subjecting the hydrogenation products to cyclization in the presence of a solid acid catalyst, whereby the intermediate present therein is converted into (S)-β-hydroxy-γ-butyrolactone; and
d) recovering said (S)-β-hydroxy-γ-butyrolactone from the resulting products,
wherein R represents linear or cyclic alkyls, or aryl groups, of from 1 to 10 carbon atoms, and R' represents hydrogen or methyl.

### BRIEF DESCRIPTION OF THE DRAWING

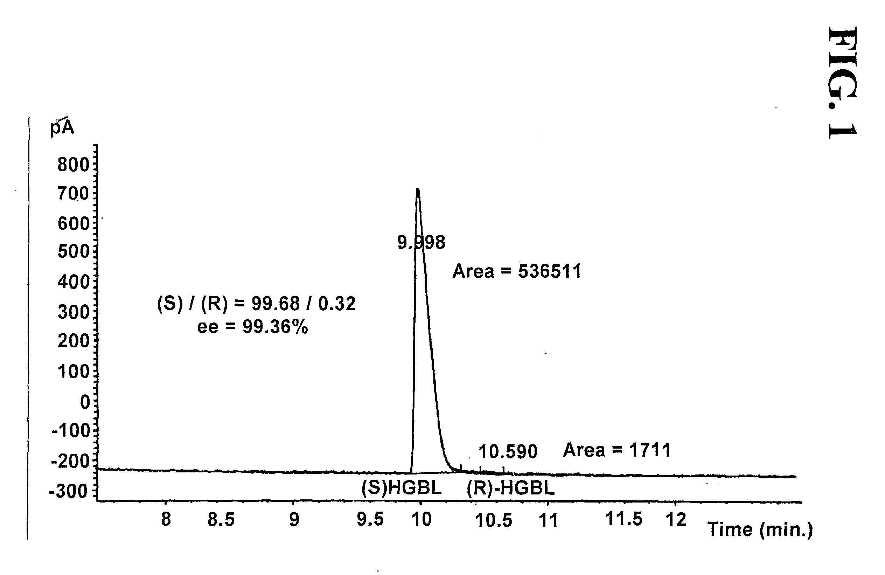

Fig. 1 is a graph showing an optical purity of (S)-β-hydroxy-γ-butyrolactone prepared by hydrogenating carboxylic acid ester derivative according to the present invention, measured with gas chromatography (GC) (Example 11).

### BEST MODES FOR CARRYING OUT THE INVENTION

According to the present invention, optically pure (S)-β-hydroxy-γ-butyrolactone is continuously produced through hydrogenation of carboxylic acid ester derivative while continuously passing it through a fixed bed reactor charged with -a catalyst comprising a metal-impregnated support. This process is advantageous in that higher yield of a target product can be achieved while retaining the optical purity to a desired level, and in that regeneration and continuous use of catalyst are assured. Further, it is not required to perform a complicated post-treatment step such as removal of the catalyst using a filter.

The present continuous process for the production of (S)-β-hydroxy-γ-butyrolactone is represented in the following Reaction Scheme 1. Carboxylic acid 1 is converted in the presence of a solid acid catalyst into carboxylic acid ester derivative 2, which is then dissolved in a solvent added with an organic or inorganic acid. The solution is fed to a fixed bed reactor, and the carboxylic acid ester derivative 2 contained therein is subjected to hydrogenating in the presence of a catalyst having a metal component highly dispersed on an inorganic oxide support. As a result, (S)-β-hydroxy-γ-butyrolactone 4, a target product, may be directly produced, or a reaction intermediate such as methyl-di-hydroxy-γ-butyric acid ester 3 may be formed. At the same time, the carboxylic acid ester derivative 2, which is a reactant, may be partly hydrolyzed and converted into carboxylic acid 1 again.

After hydrogenation, such a re-formed carboxylic acid 1, which is present in the resulting reaction products, may be removed through esterification by use of alcohol in the presence of an acid catalyst. In addition, the reaction intermediate such as methyl-di-hydroxy-butyric acid ester 3 may be converted into optically pure (S)-β-hydroxy-γ-butyrolactone 4 through cyclization reaction without performing separation in advance. 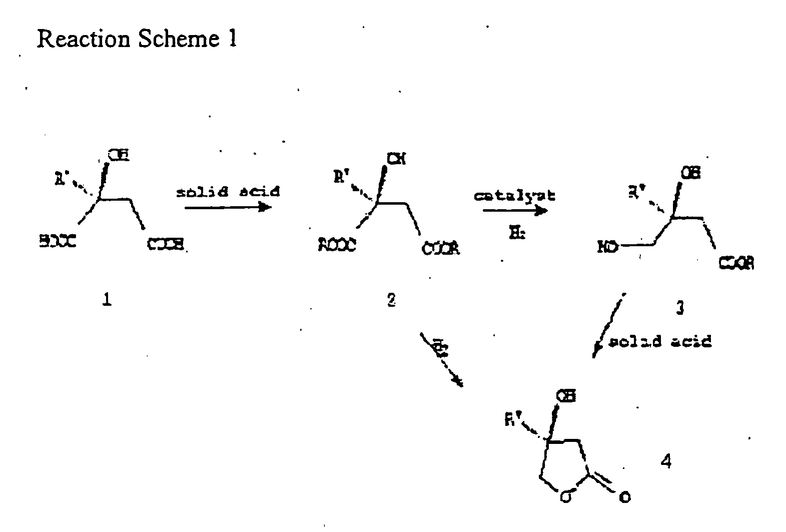 wherein R represents linear or cyclic alkyls, or aryl groups; of from 1 to 10 carbon atoms, and R' represents hydrogen or methyl.

The metal component in the hydrogenation catalyst suitable in the present invention may be selected from the group consisting of nickel (Ni), palladium (Pd), platinum (Pt), rhodium (Rh), iridium (Ir), ruthenium (Ru), osmium (Os), and combinations thereof. As a support, use can be made of alumina, silica, silica-alumina, zirconia, titania, zeolite or a molecular sieve.

The carboxylic acid ester derivative 2, which is used for the preparation of optically pure (S)-β-hydroxy-γ-butyrolactone as a starting material, is obtained through esterification of the carboxylic acid with an alcohol that is exemplified by linear alcohols, such as methyl alcohol, ethyl alcohol, n-propyl alcohol, etc., cyclic or aromatic alcohols, having 1-10 carbon atoms. The alcohol component is preferably used at an amount of 2-40 equivalents based on the carboxylic acid 1. In the solid acid catalyst, the esterification is preferably performed at 50-150 °C under a pressure of 0.069-20.7 bar (1-300 psig) at weight-hourly-space-velocity (WHSV) of 0.1-10 h⁻¹. As such, the solid acid catalyst is preferably a sulfonate-substituted resin having strong acidity. Further, L-malic acid or L-citramalic acid may be used as the carboxylic acid 1.

If the reaction is carried out beyond the above conditions, a yield of the carboxylic acid ester derivative 2 may be decreased and the deactivation rate of the catalyst is increased, thus lowering the advantages of a continuous production.

The hydrogenation of the carboxylic acid ester derivative 2 for the production of the substituted γ-butyrolactone is performed at 50-500 °C under a hydrogen partial pressure of 1.0-379.2 bar (15-5,500 psig) at weight-hourly-space-velocity (WHSV) of 0.1-10 h⁻¹, and preferably, at 60-250 °C under 68.95-344.7 bar (1,000-5,000 psig) at WHSV of 0.2-10 h⁻¹, and more preferably, at 60-200 °C under 82.74-310.3 (1,200-4,500 psig) at WHSV of 0.2-6 h⁻¹.

If the reaction is conducted outside of the above conditions, a production yield of the substituted γ-butyrolactorne product is decreased or the deactivation rate of the catalyst is increased, thus losing the advantages of a continuous production.

In order to completely convert the carboxylic acid ester derivative 2 by hydrogenation, a molar ratio of hydrogen to carboxylic acid ester derivative should be 1.0 or higher. However, in consideration of economic benefit of the process, such a molar ratio is preferably adjusted in the range of from 2 to 10. As such, the non-reacted hydrogen, which passes through the reactor, is re-compressed and circulated to the reactor. Depending on the reaction conditions, the target product may be directly recovered from the resulting reaction mixture through separation. Alternatively, in order to further increase the conversion rate, the resulting reaction products may be recirculated to the reactor, and then the target product is recovered through separation.

In the hydrogenation, it is required to use a specific solvent to conduct a conversion of the carboxylic acid ester derivative 2 retaining its optical activity. In this regard, the solvent should be capable of dissolving the carboxylic acid ester derivative having high viscosity to smoothly supply such carboxylic acid ester derivative to the reactor. In addition, the solvent is responsible for easy removal of reaction heat generated during the subsequent hydrogenation (and esterification), and should not react with the reactants including the carboxylic acid ester derivative 2 and hydrogen.

In consideration of the above, the solvent required for the hydrogenation is selected from the group consisting of methyl alcohol, ethyl alcohol, n-propyl alcohol, isopropyl alcohol, dioxane, γ-butyrolactone, tetrahydrofuran, water, and combinations thereof. It is preferred that the carboxylic acid ester derivative in the solvent has a concentration of 2-50 wt%.

Meanwhile, according to the prior art literatures regarding oxidative dehydrogenation, it is reported that oxidation (dehydrogenation) does not occur under a strong acidic condition (Coord. Chem. Rev. 1999, 187, 121). However, the carboxylic acid ester derivative used in the present invention contains a secondary alcohol moiety therein, and thus during hydrogenation by metal catalyst, partial racemization occurs by oxidation (dehydrogenation) and reduction (hydrogenation) of the secondary alcohol moiety, thereby decreasing optical purity. With the aim of overcoming the problems related to racemization, an acid is introduced to the solvent as an additive. In producing (S)-β-hydroxy-γ-butyrolactone having high optical purity (ee>99.0%), there has not been reported such employment of the acid for the hydrogenation of the carboxylic acid ester derivative from any prior art literatures.

That is, the optical purity of an initial product can be increased in a continuous hydrogenation process by adding an organic or inorganic acid additive to the solvent as aforementioned. Such an acid is selected from the group consisting of formic acid, oxalic acid, DL-malic acid, acetic acid, nitric acid, sulfuric acid, phosphoric acid, hydrochloric acid, and combinations thereof. Among them, formic acid, oxalic acid or nitric acid is preferably used. The acid is used at an amount of 0.1-50 wt%, and preferably at an amount of 0.1-20 wt%, based on the solvent weight. Further, the acid may be previously diluted in water to have a desired degree of acidity. If the amount of the acid falls out of the above range, the optical purity of the substituted γ-butyrolactone product is decreased, and the deactivation rate of the catalyst is increased by impurities (e.g., sulfur) contained in the acid additive.

The catalyst used in hydrogenation is used in the form of metal-impregnated support, in which the usable metal is exemplified by nickel (Ni), palladium (Pd), platinum (Pt), rhodium (Rh), iridium (Ir), ruthenium (Ru), osmium (Os), and combinations thereof. Among them, ruthenium is most preferable. For the preparation of a ruthenium catalyst, a precursor in the form of chlorides, nitrides or acetyl acetonates thereof may be used. In order to properly control a concentration and a degree of dispersion of the used metal, chlorides and acetyl acetonates are most preferable. As the support, suitable is inorganic oxides such as alumina, silica, silica-alumina, zirconia, titania, zeolite or a molecular sieve. In particular, silica is most preferable. Silica useful as the support is 100 m²/g or more, and preferably 200-600 m²/g in a surface area measured by BET method of nitrogen adsorption, to increase a degree of dispersion of the metal.

The degree of dispersion of the metal onto the support ranges from 2 to 50%, and more preferably from 2 to 25%. If the degree is less than 2%, catalytic activity is decreased. On the contrary, the degree exceeding 50% leads to decrease of optical purity.

The shapes of the support particulates include, but are not limited to, circular, cylindrical, and granular forms. For exhibiting suitable mechanical properties, the support formed to the circular or cylindrical shape is preferably used.

The metal in the catalyst is used at an amount of 0.1-15 wt%, and more preferably, 0.5-10 wt%. When the amount is less than 0.1 wt%, activity for hydrogenation is decreased as well as selectivity. On the other hand; when the amount exceeds 15 wt%, a process cost becomes higher due to use of expensive metals. The metal is impregnated onto the support by any conventional method such as incipient wetness impregnation, excess water impregnation, spraying or physical mixing. After impregnating the metal onto the support, calcination is conducted for 2 hours or more under an air or an inert gas atmosphere. As such, a calcination temperature should be within the range of 300-700 °C, and more preferably 300-600 °C. If the calcining temperature is lower than 300 °C, the precursor decomposition of the metal upon impregnation may become insufficient, thus resulting in incomplete calcination. Meanwhile, if the temperature is higher than 600 °C, satisfactory catalytic performance may not be exhibited due to low degree of dispersion of the metal.

After the calcined catalyst is charged into the fixed bed reactor, the catalyst is preferably reduced by hydrogen. As such, the reduction may be performed at 50-500 °C for at least 2 hours, depending on kinds of the impregnated metal.

As mentioned above, the present invention is directed to the continuous production process of optically pure substituted γ-butyrolactone with a high yield, in which the hydrogenation of carboxylic acid ester derivative compound 2 is conducted in the presence of the catalyst system of the metal-impregnated support. This process is advantageous in terms of higher yield of the target product due to adopting continuous reaction by use of a fixed bed reactor, economic benefit due to use of easily regenerable catalyst, and simplicity of the recovery process requiring no removal step of the catalyst with a filter.

In the present invention, the fixed bed reaction system is adopted to perform the process as described above. Although the fixed bed reaction system is not limited to specific reactor types or addition and flowing directions of reactants, it is preferable to use a trickle-bed type reactor equipped with an apparatus capable of uniformly dispersing hydrocarbons and hydrogen as the reactants in the whole reaction system while the reactants flow from the upper portion to the lower portion in the reactor so as to smoothly come in contact with the reactants.

The resulting reaction mixture discharged from the reactor flows to a solvent recovery apparatus, in which at least a portion of the solvent is separated from the reaction product. For example, the solvent recovery can be accomplished by a distillation tower and a flash vaporizer. The product or concentrated material discharged from the lower portion of the solvent recovery apparatus is transferred to a vacuum distillation apparatus.

According to the present invention, an improved catalyst is employed for the production of (S)-β-hydroxy-γ-butyrolactone. Reaction yield and productivity may be increased by use of such a catalyst. Moreover, introduction of the inorganic or organic acid additive results in production of (S)-β-hydroxy-γ-butyrolactone having excellent optical purity by a continuous hydrogenation process.

A better understanding of the present invention may be obtained in light of the following examples which are set forth to illustrate, but are not to be construed to limit the present invention.

### EXAMPLE 1

### Preparation of Catalyst

To a 500 cc flask, secondary distilled water and then 18 g of ruthenium chloride (RuCl₂) were introduced, yielding an aqueous ruthenium chloride solution. Circular silica 100 g (1/8") was added to a rotatable metal impregnation container equipped with a motor capable of controlling a rotational speed, and then the ruthenium solution was uniformly dispersed to the silica while the container was rotated. Even after addition of the ruthenium solution was terminated, the motor was further rotated at the same rotational speed for about 60 minutes. Then, the ruthenium-impregnated silica was transferred to a muffle furnace and calcined at 500 °C for 3 hours under an air atmosphere. The content of ruthenium in the sintered catalyst, measured by X-ray fluorescence analysis, was 3.0 wt%.

### EXAMPLE 2

### Continuous Preparation of Dimethyl (S)-malate

25 g of a solid acid catalyst was charged into an automated high pressure fixed bed reactor made of 316 stainless steel, and then purged with nitrogen gas. Thereafter, the temperature in the reactor was raised from room temperature to 105 °C and the reaction pressure was maintained at 6.89 bar (100 psig). Then, L-malic acid was dissolved in 8 equivalents of methyl alcohol, and was introduced into the reactor at WHSV 4.0 h⁻¹, yielding a reaction product with a conversion efficiency of 93%, a reaction selectivity of 98% and a yield of 91%.

The resulting reaction mixture by the continuous manner as above was distilled under reduced pressure, to obtain dimethyl (S)-malate having purity of 99.8%, optical purity of 99.9% or more and an isolation yield of 90%. This product might be afforded even by a batch type reaction requiring a reaction time period of 2-4 hours.

### EXAMPLES 3-6

### Continuous Preparation of (S)-β-hydroxy-γ-butyrolactone

10 g of the catalyst prepared by the method of Example 1 was charged into a continuous high pressure reactor made of 316 stainless steel. After the temperature in the reactor was raised to 350 °C at a rate of 2 °C/minute, the catalyst was reduced under a hydrogen atmosphere for 6 hours, and then the reactor cooled to room temperature was purged with nitrogen gas. While the temperature in the reactor was raised from room temperature to a reaction temperature at a rate of 1 °C/minute, hydrogen of 100 sccm was introduced thereto. The amount of the introduced hydrogen was twice the amount required for reaction, and dimethyl-(S)-malate was dissolved in water added with an acid additive. The reactant solution having 20 wt% of dimethyl (S)-malate was fed to the reactor and hydrogenated at 95-125 °C under a hydrogen pressure of 233.0 bar (3,380 psig) at WHSV 1.5 h. In case of using water containing an acid additive (*), a desired optical purity was confirmed at the initial reaction stage (6 hour after the reaction was initiated). The results are shown in Table 1, below. In case of using water not added with an acid, it was not until 50 hours later that ee value of 99% or more was obtained. The reaction product was analyzed with a FID (flame ionization detector) after gas chromatography (30m×0.25mm×0.25m HP-5 column), and ee value thereof was measured by gas chromatography (30m×0.25mm×0.25m beta-DEX column).

**TABLE 1**

| Ex.No. | Solvent System | Reaction Temp.(°C) | Time²⁾ | Conversion (%) | Selectivity¹⁾, (%) | ee (%) |
|---|---|---|---|---|---|---|
| 3 | Water+A* | 110 | 6 | 77.2 | 89.2 | 99.29 |
| 4 | Water+B* | 110 | 7 | 75.4 | 90.2 | 99.27 |
| 5 | Water+C* | 115 | 5 | 70.2 | 92.2 | 99.25 |
| 6 | Water | 110 | 6 | 75.4 | 77.9 | 98.12 |

| | | | | | | |
|---|---|---|---|---|---|---|
| Note: 1) Selectivity refers to the sum of respective selectivity for methyl-di-hydroxy-butyric acid ester as a reaction intermediate and (S)-β-hydroxy-γ-butyrolactone; and 2) Time period from the beginning of the reaction to the collection of the sample A*: formic acid, B*: oxalic acid, C*: nitric acid. | | | | | | |

### EXAMPLES 7-10

In hydrogenation of dimethyl (S)-malate using the solvent such as alcohol or water mixed with 2% formic acid additive, the higher optical purity was assured in a. water than in alcohol. These examples were carried out in the manner set forth in Example 3 under the conditions of temperature of 100-135 °C, hydrogen pressure of 233.0 bar (3,380 psig) and WHSV 1.5 h⁻¹. The used solvent and the reaction results are given in the following Table 2.

**TABLE 2**

| Ex.No.¹⁾ | Temp. (°C) | Solvent | Conversion (%) | Selectivity²⁾ (%) | ee (%) |
|---|---|---|---|---|---|
| 7 | 120 | H₂O | 79.2 | 88.1 | 99.19 |
| 8 | 125 | MeOH | 83.0 | 84.9 | 98.25 |
| 9 | 125 | EtOH | 85.7 | 84.1 | 97.52 |
| 10 | 125 | IPA | 95.2 | 43.5 | 96.12 |

| | | | | | |
|---|---|---|---|---|---|
| Note: 1) catalyst: Example 1; and 2) Selectivity refers to the sum of respective selectivity for methyl-di-hydroxy-butyric acid ester as a reaction intermediate and (S)-β-hydroxy-γ**-**butyrolactone. | | | | | |

### EXAMPLES 11-13

### Continuous Preparation of (S)-β-hydroxy-γ-butyrolactone

Hydrogenation of dimethyl (S)-malate was carried out in the manner set forth in Example 3 under the conditions of temperature of 110 °C, hydrogen pressure of 233.0 bar (3,380 psig) and WHSV 0.5-1.5 varying with kinds of the catalyst, in the water solvent system added with formic acid. In the following Table 3, the results show the influence of the degree of dispersion of the metal in terms of the production efficiency. The degree of dispersion, measured with chemical adsorption of carbon monoxide, refers to a value calculated from the equation (molecules of carbon monoxide adsorbed to a metal atom × 100). As shown in Table 3, when the degree of dispersion is increased, a space-time yield is drastically increased with maintenance of ee value.

**TABLE 3**

| Ex.No. | Catalyst System | Dispersion (%) | WHSV (h⁻¹) | Conversion (%) | Selectivity¹⁾ (%) | ee (%) |
|---|---|---|---|---|---|---|
| 11 | Ru/SiO₂-A | 18 | 1.5 | 79.4 | 82.3 | 99.36 |
| 12 | Ru/SiO₂-B | 4.0 | 1.0 | 76.5 | 82.5 | 99.20 |
| 13 | Ru/SiO₂-C | 1.0 | 0.5 | 85.4 | 84.2 | 99.18 |

| | | | | | | |
|---|---|---|---|---|---|---|
| Note: 1) Selectivity refers to the sum of respective selectivity for methyl-di-hydroxy-butyric acid ester as a reaction intermediate and (S)-β-hydroxy-γ-butyrolactone. | | | | | | |

### EXAMPLE 14

Hydrogenation of dimethyl (S)-malate was carried out in the manner set forth in Example 3 under the conditions of temperature of 100 °C, and WHSV 1.5 h⁻¹ in the presence of Ru/SiO₂-A catalyst employed in Example 11 using a water solvent system added with formic acid. The reaction results varying with the applied hydrogen pressure are given in the following Table 4.

**TABLE 4**

| Pressure | Conversion (%) | Selectivity¹⁾ (%) | ee (%) |
|---|---|---|---|
| 171.2 bar (2,483 psig) | 65.1 | 85.2 | 99.19 |
| 233.0 bar (3,380) psig) | 74.0 | 83.0 | 99.36 |

| | | | |
|---|---|---|---|
| Note: 1) Selectivity refers to the sum of respective selectivity for methyl-di-hydroxy-buyric acid ester as a reaction intermediate and (S)-β-hydroxy-γ-butyrolactone. | | | |

### EXAMPLES 15-22

Hydrogenation of dimethyl (S)-malate was carried out in the manner set forth in Example 3. At this time, the solvent and reaction conditions were variously changed without use of the acid additive. When the acid additive was not used, the optical purity was lowered, compared with the case of using the acid additive. The reaction results are given in the following Table 5.

**TABLE 5**

| Ex.No.¹⁾ | Solvent | Temp. (°C) | WHSV (h⁻¹) | Conversion (%) | Selectivity²⁾ (%) | ee (%) |
|---|---|---|---|---|---|---|
| 15 | MeOH | 120 | 0.5 | 75.2 | 85.6 | 98.10 |
| 16 | MeOH | 135 | 0.5 | 84.2 | 78.4 | 97.56 |
| 17 | H₂O | 120 | 1.0 | 74.2 | 79.3 | 98.15 |
| 18 | H₂O | 135 | 1.0 | 84.0 | 75.5 | 97.25 |
| 19 | IPA | 135 | 0.25 | 97.2 | 42.1 | 95.32 |
| 20 | THF | 130 | 0.5 | 75.6 | 84.0 | 97.23 |
| 21 | EtOH | 125 | 0.5 | 86.1 | 55.2 | 96.52 |
| 22 | EtOH | 125 | 1.0 | 79.8 | 64.6 | 97.12 |

| | | | | | | |
|---|---|---|---|---|---|---|
| Note: 1) catalyst: Example 1 2) Selectivity refers to the sum of respective selectivity for methyl-di-hydroxy-butyric acid ester as a reaction intermediate and (S)-β-hydroxy-γ-butyrolactone. | | | | | | |

### EXAMPLE 23

### Continuous Reaction of (S)-β-hydroxy-γ-butyrolactone for Long-term Period

Using the catalyst prepared in Example 1, a continuous reaction was carried out in a reactor similar to that illustrated in Example 3 for a long-term period. Even though the reaction was carried out for 600 hours or more, no deactivation of the catalyst was observed. Such results are presented in Table 6, below.

**TABLE 6**

| Reaction Time | 100 hr | 200 hr | 600 hr |
|---|---|---|---|
| Conversion (%) | 75.2 | 74.1 | 73.9 |
| Selectivity¹⁾ (%) | 83.5 | 84.2 | 85.2 |
| Optical Purity (%) | 99.17 | 99.21 | 99.23 |

| | | | |
|---|---|---|---|
| Note: 1) Selectivity refers to the sum of respective selectivity for methyl-di-hydroxy-butyric acid ester as a reaction intermediate and (S)-β-hydroxy-γ-butyrolactone. | | | |

### EXAMPLE 24

### Production and Isolation of (S)-β-hydroxy-γ-butyrolactone

Using 50 g of the catalyst prepared in Example 1, the hydrogenation reaction was carried out in a reactor similar to that illustrated in Example 3. During the reaction, while pressure was maintained to 233.0 bar (3,380 psig) the reaction temperature and WHSV were variously changed. After the reaction was carried out for 200 hours, 30 liters of the solution containing (S)-β-hydroxy-γ-butyrolactone with a selectivity of 84.1 % was obtained. In order to recover (S)-β-hydroxy-γ-butyrolactone, the solution was concentrated to remove the solvent, and the acid present therein was removed through esterification using alcohol in the presence of an acid catalyst Subsequently, methyl-di-hydroxy-butyric acid ester as a reaction intermediate was cyclized in the presence of the acid catalyst without any isolation. At this time, the cyclization was carried out with no addition of solvent. The resulting reaction product was then repeatedly extracted 3 times with chloroform. The extracted material was introduced into a glass reactor of 10 liter volume equipped with a vacuum distillator, vacuum distilled at 60 °C under 100 mbar to remove the solvent, and then concentrated. Using a thin film evaporator, the concentrated product was further vacuum distilled at 100-120 °C under 0.6-1.7 torr. As a result, (S)-β-hydroxy-γ-butyrolactone having an isolation yield of 65%, purity of 98.00%, and optical purity of 99.32% was obtained.

### INDUSTRIAL APPLICABILITY

As described above, according to the production process of the preset invention, chemically pure (S)-β-hydroxy-γ-butyrolactone having desired optical activity can be obtained by hydrogenating the carboxylic acid ester derivative in the presence of the catalyst system comprising metal-impregnated support. Therefore, the inventive process is advantageous in light of higher purity, higher optical purity and higher yield of the desired product, and relatively simpler and environmentally safer process than conventional processes. In addition, the desired product can be produced on a large scale due to increased production efficiency.

## Claims

1. A continuous process for the production of chemically pure (S)-β-hydroxy-γ-butyrolactone having desired optical activity, which comprises:
a) dissolving carboxylic acid ester derivative having the following Formula 2 in solvent at an amount of 2-50 wt%, the solvent being added with an organic or inorganic acid; 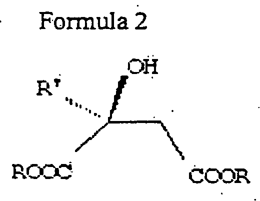
b) subjecting the carboxylic acid ester derivative solution to hydrogenation at 50-500 °C under a pressure of 1.0-379.2 bar (15-5,500 psig) at weight-hourly-space-velocity of 0.1-10 h⁻¹, in a fixed bed reactor charged with a metal catalyst-impiegnated inorganic oxide support, a molar ratio of hydrogen to carboxylic acid ester derivative ranging from 2 to 10; wherein the metal catalyst is selected from the group consisting of nickel (Ni), palladium (Pd), platinum (Pt), rhodium (Rh), iridium (Ir), ruthenium (Ru), osmium (Os), and combinations thereof; and
c) recovering (S)-β-hydroxy-γ-butyrolactone having the following Formula 4 from the hydrogenation products, 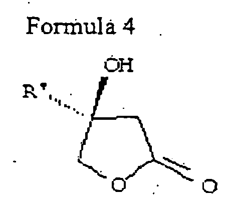
wherein R represents linear or cyclic alkyls, or aryl groups, of from 1 to 10 carbon atoms, and R' represents hydrogen or methyl.

2. A continuous process for the production of chemically pure (S)-β-hydroxy-γ-butyrolactone having desired optical activity, which comprises:
a) dissolving carboxylic acid ester derivative having the following Formula 2 in solvent at an amount of 2-50 wt%, the solvent being added with an organic or inorganic acid; 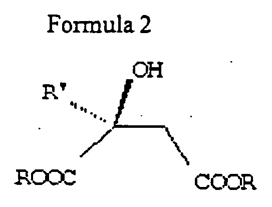
b) subjecting the carboxylic acid ester derivative solution to hydrogenation at 50-500 °C under a pressure of 1.0-379.2 bar (15-5,500 psig) at weight-hourly-space-velocity of 0.1-10 h⁻¹, in a fixed bed reactor charged with a metal catalyst-impregnated inorganic oxide support to give hydrogenation products containing an intermediate having the following Formula 3 or (S)-β-hydroxy-γ-butyrolactone having the following Formula 4, a molar ratio of hydrogen to carboxylic acid ester derivative ranging from 2 to 10 wherein the metal catalyst is selected from the group consisting of nickel (Ni), palladium (Pd), platinum (Pt), rhodium (Rh), iridium (Ir), ruthenium (Ru), osmium (Os), and combinations thereof; and 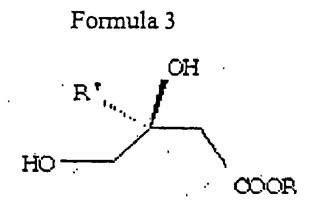 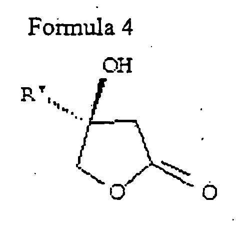
c) subjecting the hydrogenation products to cyclization in the presence of a solid acid catalyst, whereby the intermediate present therein is converted into (S)-β-hydroxy-γ-butyrolactone; and
d) recovering said (S)-β-hydroxy-γ-butyrolactone from the resulting products,
wherein R represents linear or cyclic alkyls, or aryl groups, of from 1 to 10 carbon atoms, and R' represents hydrogen or methyl.

3. The process as defined in claim 1 or 2, wherein the metal catalyst is impregnated at an amount of 0.1-15 wt%.

4. The process as defined in either claim 1 or claim 2, wherein the metal catalyst is ruthenium (Ru).

5. The process as defined in claim 1 or 2, wherein a degree of dispersion of the metal in the catalyst is adjusted in the range of 2-50%.

6. The process as defined in claim 1 or 2, wherein the hydrogenation step is performed at 60-200 °C.

7. The process as defined in claim 1 or 2, wherein the hydrogenation step is performed under a pressure of 82.74-310.3 bar (1,200-4,500 psig).

8. The process as defined in claim 1 or 2, wherein the hydrogenation step is carried out at weight-hourly-space-velocity of 0.2-6.0 h⁻¹.

9. The process as defined in claim 1 or 2, wherein the organic or inorganic acid additive in the solvent is added at an amount of 0.11 -20 wt%, based on the solvent weight

10. The process as defined in claim 1 or 2, wherein the solvent is selected from the group consisting of methyl alcohol, ethyl alcohol, n-propyl alcohol, isopropyl alcohol, dioxane, γ-butyrolactone, tetrahydrofuran, water, and combinations thereof.

11. The process as defined in claim 1 or 2, wherein the acid additive is selected from the group consisting of formic acid, oxalic acid, nitric acid, DL-malic acid, acetic acid, sulfuric acid, phosphoric acid, hydrochloric acid, and combinations thereof.

12. The process as defined in claim 1 or 2, wherein the inorganic oxide support is selected from the group consisting of alumina, silica, silica-alumina, zirconia, titania, zeolite and a molecular sieve.

13. The process as defined in claim 1 or 2, wherein the carboxylic acid ester derivative is obtained by reacting carboxylic acid with a linear, cyclic or aromatic alcohol having 1-10 carbon atoms in the presence of a solid acid catalyst, under conditions of a temperature of 50-150 °C, a pressure of 0.069-20.7 bar (1.0-300 psig) and weight-hourly-space-velocity of 0.1-10 h⁻¹, in which the alcohol is used at an amount of 2.0-40 equivalents based on the carboxylic acid.

14. The process as defined in claim 13, wherein said carboxylic acid is L-malic acid or L-citramalic acid.

15. The process as defined in claim 2, further comprising removing carboxylic acid of Formula 1 through esterification with alcohol in the presence of an acid catalyst prior to the step c), 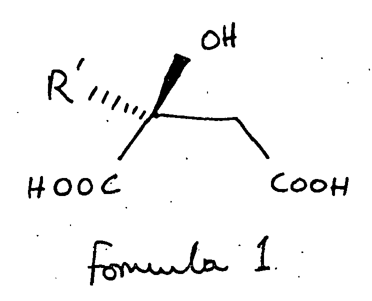 wherein R' represents hydrogen or methyl.

## Patentansprüche

1. Kontinuierliches Verfahren zur Herstellung von chemisch reinem (S)-β-Hydroxy-γ-butyrolacton mit gewünschter optischer Aktivität, das die folgenden Schritte beinhaltet:
a) Auflösen von Carbonsäureesterderivat mit der folgenden Formel 2 in einem Lösungsmittel in einer Menge von 2-50 Gew.-%, wobei das Lösungsmittel mit einer organischen oder anorganischen Säure zugegeben wird: 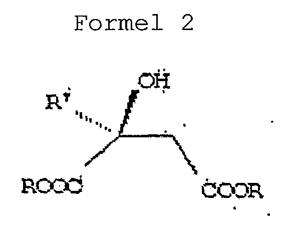
b) Hydrieren der Carbonsäureesterderivatlösung bei 50-500°C unter einem Druck von 1,0 - 379,2 bar (15 - 5500 psig) mit einer massenbezogenen Raumgeschwindigkeit (WHSV) von 0,1 - 10 h⁻¹ in einem Festbettreaktor, der mit einer mit einem Metallkatalysator imprägnierten anorganischen Oxidunterlage beschickt ist, einem Molverhältnis von Wasserstoff zu Carbonsäureesterderivat im Bereich von 2 bis 10, wobei der Metallkatalysator aus der Gruppe bestehend aus Nickel (Ni), Palladium (Pd), Platin (Pt), Rhodium (Rh), Iridium (Ir), Ruthenium (Ru), Osmium (Os) und Kombinationen davon ausgewählt ist; und
c) Zurückgewinnen des (S)-β-Hydroxy-γ-butyrolactons mit der folgenden Formel 4 aus den Hydrierungsprodukten, 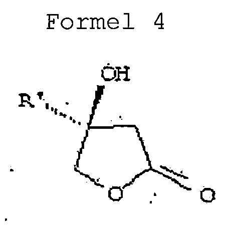
wobei R lineare oder cyclische Alkyle oder Arylgruppen mit 1 bis 10 Kohlenstoffatomen und R' Wasserstoff oder Methyl repräsentieren.

2. Kontinuierliches Verfahren zur Herstellung von chemisch reinem (S)-β-Hydroxy-γ-butyrolacton mit gewünschter optischer Aktivität, das die folgenden Schritte beinhaltet:
a) Auflösen von Carbonsäureesterderivat mit der folgenden Formel 2 in einem Lösungsmittel in einer Menge von 2-50 Gew.-%, wobei das Lösungsmittel mit einer organischen oder anorganischen Säure zugegeben wird; 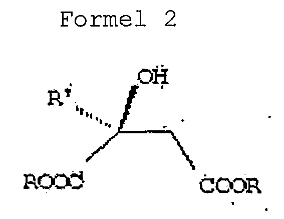
b) Hydrieren der Carbonsäureesterderivatlösung bei 50-500°C unter einem Druck von 1,0 - 379,2 bar (15 - 5500 psig) mit einer massenbezogenen Raumgeschwindigkeit (WHSV) von 0,1 - 10 h⁻¹ in einem Festbettreaktor, der mit einer mit einem Metallkatalysator imprägnierten anorganischen Oxidunterlage beschickt ist, um Hydrierungsprodukte zu erhalten, die ein Intermediat mit der folgenden Formel 3 oder (S)-β-Hydroxy-γ-butyrolacton mit der folgenden Formel 4 enthalten, einem Molverhältnis von Wasserstoff zu Carbonsäureesterderivat im Bereich von 2 bis 10, wobei der Metallkatalysator aus der Gruppe bestehend aus Nickel (Ni), Palladium (Pd), Platin (Pt), Rhodium (Rh), Iridium (Ir), Ruthenium (Ru), Osmium (Os) und Kombinationen davon ausgewählt ist; und 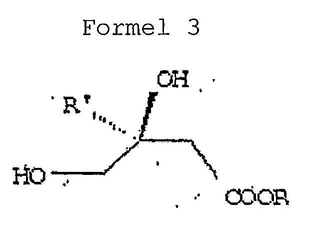 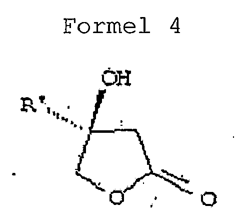
c) Cyclisieren der Hydrierungsprodukte in Anwesenheit eines festen Säurekatalysators, so dass das darin vorliegende Intermediat in (S)-β-Hydroxy-γ-butyrolacton umgewandelt wird; und
d) Zurückgewinnen des (S)-β-Hydroxy-γ-butyrolactons aus den resultierenden Produkten,
wobei R lineare oder cyclische Alkyle oder Arylgruppen mit 1 bis 10 Kohlenstoffatomen und R' Wasserstoff oder Methyl repräsentieren.

3. Verfahren nach Anspruch 1 oder 2, wobei der Metallkatalysator mit einer Menge von 0,1 - 15 Gew.-% imprägniert ist.

4. Verfahren nach Anspruch 1 oder Anspruch 2, wobei der Metallkatalysator Ruthenium (Ru) ist.

5. Verfahren nach Anspruch 1 oder 2, wobei ein Dispersionsgrad des Metalls in dem Katalysator im Bereich von 2 - 50% eingestellt wird.

6. Verfahren nach Anspruch 1 oder 2, wobei der Hydrierungsschritt bei 60 - 200°C ausgeführt wird.

7. Verfahren nach Anspruch 1 oder 2, wobei der Hydrierungsschritt unter einem Druck von 82,74 - 310,3 bar (1200 - 4500 psig) ausgeführt wird.

8. Verfahren nach Anspruch 1 oder 2, wobei der Hydrierungsschritt bei einer massenbezogenen Raumgeschwindigkeit (WHSV) von 0,2 - 6,0 h⁻¹ ausgeführt wird.

9. Verfahren nach Anspruch 1 oder 2, wobei das organische oder anorganische Säureadditiv in dem Lösungsmittel in einer Menge von 0,1 - 20 Gew.-% auf der Basis des Lösungsmittelgewichtes zugegeben wird.

10. Verfahren nach Anspruch 1 oder 2, wobei das Lösungsmittel aus der Gruppe bestehend aus Methylalkohol, Ethylalkohol, N-Propylalkohol, Isopropylalkohol, Dioxan, γ-Butyrolacton, Tetrahydrofuran, Wasser und Kombinationen davon ausgewählt wird.

11. Verfahren nach Anspruch 1 oder 2, wobei das Säureadditiv ausgewählt wird aus der Gruppe bestehend aus Ameisensäure, Oxalsäure, Salpetersäure, DL-Äpfelsäure, Essigsäure, Schwefelsäure, Phosphorsäure, Chlorwasserstoffsäure und Kombinationen davon.

12. Verfahren nach Anspruch 1 oder 2, wobei die anorganische Oxidunterlage ausgewählt ist aus der Gruppe bestehend aus Aluminiumoxid, Siliciumdioxid, Siliciumdioxid-Aluminiumoxid, Zirconiumoxid, Titandioxid, Zeolith und einem Molekülsieb.

13. Verfahren nach Anspruch 1 oder 2, wobei das Carbonsäureesterderivat durch Reagieren von Carbonsäure mit einem linearen, cyclischen oder aromatischen Alkohol mit 1 - 10 Kohlenstoffatomen in Anwesenheit eines festen Säurekatalysators unter Bedingungen von einer Temperatur von 50 - 150°C, einem Druck von 0,069 - 20,7 bar (1,0 - 300 psig) und einer massenbezogenen Raumgeschwindigkeit (WHSV) von 0,1 - 10 h⁻¹ erhalten wird, wobei der Alkohol in einer Menge von 2,0 - 40 Äquivalenten auf der Basis der Carbonsäure benutzt wird.

14. Verfahren nach Anspruch 13, wobei die genannte Carbonsäure L-Äpfelsäure oder L-Zitramalsäure ist.

15. Verfahren nach Anspruch 2, das ferner das Entfernen von Carbonsäure von Formel 1 durch Veresterung mit Alkohol in Anwesenheit eines Säurekatalysators vor Schritt c) beinhaltet: 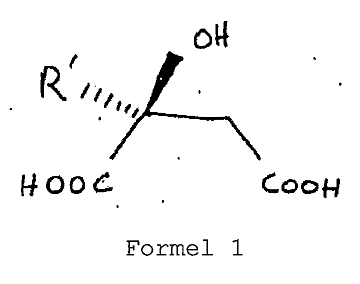 wobei R' Wasserstoff oder Methyl repräsentiert.

## Revendications

1. Procédé continu pour la production de (S)-β-hydroxy-γ-butyrolactone chimiquement pur, ayant l'activité optique souhaitée, le procédé consistant à :
a) dissoudre dans un solvant, en une quantité de 2 à 50% en poids, un dérivé d'ester d'acide carboxylique ayant la formule 2 ci-dessous, le solvant étant ajouté avec un acide organique ou inorganique ; 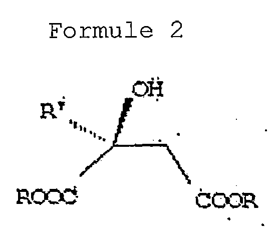
b) soumettre le dérivé d'ester d'acide carboxylique à une hydrogénation à 50 à 500°C sous une pression de 1,0 à 379,2 bars (15 à 5500 psig) à une vitesse spatiale horaire pondérale de 0,1 à 10h⁻¹, dans un réacteur à lit fixe chargé avec un support en oxyde inorganique imprégné d'un catalyseur métallique, un rapport molaire hydrogène/dérivé d'ester d'acide carboxylique compris entre 2 et 10, dans lequel le catalyseur métallique est sélectionné dans le groupe comprenant: nickel (Ni), palladium (Pd), Platine (Pt), rhodium (Rh) iridium (Ir), ruthénium (Ru), osmium (Os), et des combinaisons de ceux-ci ; et
c) récupérer, à partir des produits d'hydrogénation, le (S)-β-hydroxy-γ-butyrolactone ayant la formule 4 ci-dessous ; 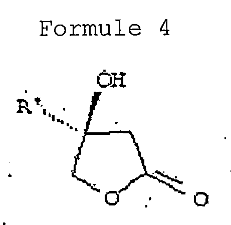
dans laquelle R représente des alkyles linéaires ou cycliques, ou des groupes aryles ayant 1 à 10 atomes de carbone, et R' représente hydrogène ou méthyle.

2. Procédé continu pour la production de (S)-β-hydroxy-γ-butyrolactone ayant l'activité optique souhaitée, le procédé consistant à :
a) dissoudre dans un solvant, en une quantité de 2 à 50% en poids, un dérivé d'ester d'acide carboxylique ayant la formule 2 ci-dessous, le solvant étant ajouté avec un acide organique ou inorganique ; 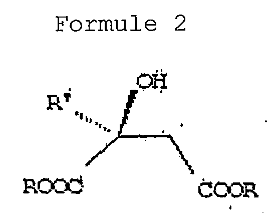
b) soumettre la solution de dérivé d'ester d'acide carboxylique à l'hydrogénation à 50 à 500°C sous une pression de 1,0 à 379,2 bars (15 à 5500 psig) à une vitesse spatiale horaire pondérale de 0,1 à 10 h⁻¹ dans un réacteur à lit fixe chargé avec un support acide inorganique imprégné d'un catalyseur métallique pour donner des produits d'hydrogénation contenant un intermédiaire ayant la formule 3 ci-dessous, ou (S)-β-hydroxy-γ-butyrolactone ayant la formule 4 ci-dessous, un rapport molaire hydrogène/dérivé d'ester d'acide carboxylique compris entre 2 et 10, dans lequel le catalyseur métallique est sélectionné dans le groupe comprenant : nickel (Ni), palladium (Pd), platine (Pt), rhodium (Rh), iridium (Ir), ruthénium (Ru), osmium (Os) et des combinaisons de ceux-ci ; et 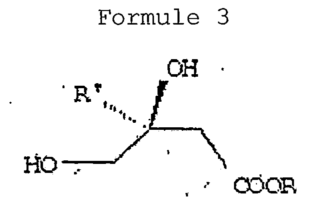 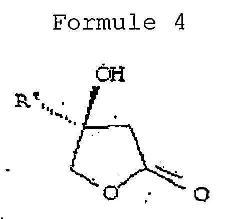
c) soumettre les produits de l'hydrogénation à une cyclisation en présence d'un catalyseur acide solide, de sorte que l'intermédiaire présent dans celui-ci est converti en (S)-β-hydroxy-γ-butyrolactone ; et
d) récupérer ledit (S)-β-hydroxy-γ-butyrolactone à partir des produits résultants, R représentant des alkyles linéaires ou cycliques, ou des groupes aryles ayant 1 à 10 atomes de carbone, et R' représentant hydrogène ou méthyle.

3. Procédé selon la revendication 1 ou 2, selon lequel le catalyseur métallique est imprégné en une quantité de 0,1 à 15% en poids.

4. Procédé selon la revendication 1 ou 2, selon lequel le catalyseur métallique est le ruthénium (Ru).

5. Procédé selon la revendication 1 ou 2, selon lequel un niveau de dispersion du métal dans le catalyseur est ajusté dans la plage de 2% à 50%.

6. Procédé selon la revendication 1 ou 2, selon lequel l'étape d'hydrogénation est réalisée à une température de 60 à 200°C.

7. Procédé selon la revendication 1 ou 2, selon lequel l'étape d'hydrogénation est réalisée sous une pression de 82,74 à 310,3 bars (1200 à 4500 psig).

8. Procédé selon la revendication 1 ou 2, selon lequel l'étape d'hydrogénation est réalisée à une vitesse spatiale horaire pondérale de 0,2 à 6,0 h⁻¹.

9. Procédé selon la revendication 1 ou 2, selon lequel l'additif acide organique ou inorganique dans le solvant est ajouté en une quantité de 0,1 à 20% en poids, en fonction du poids du solvant.

10. Procédé selon la revendication 1 ou 2, selon lequel le solvant est sélectionné dans le groupe comprenant : méthanol, éthanol, alcool propylique normal, isopropanol, dioxane, γ-butyrolactone, tétrahydrofurane, eau et combinaisons de ceux-ci.

11. Procédé selon la revendication 1 ou 2, selon lequel l'additif acide est sélectionné dans le groupe comprenant: acide formique, acide oxalique, acide nitrique, acide malique DL, acide acétique, acide sulfurique, acide phosphorique, acide chlorhydrique et combinaisons de ceux-ci.

12. Procédé selon la revendication 1 ou 2, selon lequel l'acide inorganique du support est sélectionné dans le groupe comprenant : alumine, silice, silice-alumine, zirconiums, titanes, zéolite et un tamis moléculaire.

13. Procédé selon la revendication 1 ou 2, selon lequel le dérivé d'ester d'acide carboxylique est obtenu en faisant réagir un alcool linéaire, cyclique ou aromatique ayant 1 à 10 atomes de carbone en présence d'un catalyseur acide solide, dans les conditions suivantes : température de 50 à 150°C, pression de 0,069 à 20,7 bars (1,0 à 300 psig) et vitesse spatiale horaire pondérale de 0,1 à 10h⁻¹, vitesse à laquelle l'alcool est utilisé en une quantité de 2,0 à 40 équivalents basés sur l'acide carboxylique.

14. Procédé selon la revendication 13, selon lequel ledit acide carboxylique est l'acide malique L ou l'acide citramalique L.

15. Procédé selon la revendication 2, qui comprend de plus, avant l'étape c), l'extraction de l'acide carboxylique de la Formule 1 par estérification à l'alcool en présence d'un catalyseur acide. 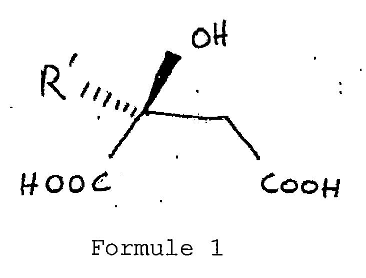 dans laquelle R' représente hydrogène ou méthyle.
